## Europäisches Patentamt

(19) **European Patent Office** ¦

Office européen des brevets

(11) Publication number: · **0 101 685**
**B1**

(12) ## EUROPEAN PATENT SPECIFICATION

(45) Date of publication of patent specification: **30.07.86**

(51) Int. Cl.⁴: **A 61 K 31/195,** A 61 K 31/70, A 61 K 37/02, A 61 K 9/08

(21) Application number: **82903595.5**

(22) Date of filing: **08.12.82**

(86) International application number: **PCT/RO82/00007**

(87) International publication number: **WO 83/02893 01.09.83 Gazette 83/20**

(54) MEDICINAL COMPOSITION FOR THE TREATMENT OF CERTAIN NEUROVIROSES.

(30) Priority: **23.02.82 RO 106709**

(43) Date of publication of application: **07.03.84 Bulletin 84/10**

(45) Publication of the grant of the patent: **30.07.86 Bulletin 86/31**

(84) Designated Contracting States: **CH DE FR GB LI**

(56) References cited:
DE-A-2 913 578
DE-B-1 069 829
DE-C- 687 563
DE-C- 739 912
FR-A-1 005 491
FR-A-1 438 158
FR-A-2 225 155
GB-A-1 550 706

**The file contains technical information submitted after the application was filed and not included in this specification**

(73) Proprietor: **CENTRALA INDUSTRIALA DE MEDICAMENTE, COSMETICE COLORANTI SI LACURI**
**Bd.Ion Sulea nr.246 Sector 3**
**R-74585 Bucuresti (RO)**

(72) Inventor: **DINU, Romulus Constantin**
**Strada Matei Voievod nr. 6A**
**Sector 2 Bucuresti (RO)**
Inventor: **DINU, Ileana Dana**
**Strada Matei Voievod, Nr. 6A**
**Sector 2 Bucuresti (RO)**

(74) Representative: **Reinhard, Skuhra, Weise**
**Leopoldstrasse 51**
**D-8000 München 40 (DE)**

(56) References cited:

**AUSTRIA CODEX 1981 1982, Vienna 1981 (EUCALICIUMPREPARATIONS)**
**La Santé Publique Revue Internationale, 1984, XXVII, pp. 99-114**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention refers to a pharmaceutical composition in the form of an aqueous injectable solution for the treatment of a neuroviruses selected from Herpes Zoster, Herpes Simplex, encephalomyelitis, polyradiculoneuritis, cranial nerve paralysis, or of the autoimmune disease — multiple sclerosis — as well as for the treatment of certain types of dementia, porphyria and Wilson's disease.

Background Art

Certain pharmaceutical preparations are known to be effective in herpes Zoster: the antibiotic Rifamycin or the immuno stimulating agent Isoprinosine (Lesourd B, Loude J, Meunier P, Doumero P, Moulias R., — Traitement du zona ZOSTER par isoprinosine — La nouv. Presse Medicale, le 23 janvier 1982 II nr.3, p.191) and other chemotherapic compounds such as idoxuridine (Juel Jensen B.E. — Treatment of Zoster with idoxuridine in dimethylsulfoxide: result of two blind control trials Br.Med.J.197 (4), p.776-780) cytosine arabinoside (Pierle L.E. — Cytosine arabinoside for herpes Zoster — N. Engl. J. Med. 1974, 290 p.404—410) and adenine arabinoside (Whitley R.J. et al — Adenine arabinoside therapy for herpes Zoster — N. Engl. J. Med. 1976—294, p.1193—1199).

Isoprinosine has a slow curative action upon Zoster eruption; its effect upon pains is practically insignificant. The other substances — idoxuridine, cytosine arabinoside and adenine arabinoside are contraindicated on account of side effects.

The antivirotic action of several classes of chelators has been ascertained against a large spectrum of viruses (Perrin D.D. and Stünzi H., 1981 — Pharmac. Ther. 22, 255) in *in vitro* studies or in animal studies; even certain compounds of the class of thiosemicarbazones were proved to be active in the prophylaxis of chicken pox in an epidemic in Madras. In our country, Gh.D. Grigorescu wrote (in 1955) that dimercapto-propanol (DMP; BAL) is effective in herpes Zoster and in some other viruses (1973); however, DMP is not indicated for humans owing to the fact that the therapeutic dose is very close to the toxic dose (L. Goodman, The pharmacological basis of therapeutics, 1980 Ed. L. Goodman, A. Gilman).

The detoxifying action of the ethylenediaminotetracetic acid is well-known; this product is used as an antidote in poisonings with bi and trivalent metals; its mechanism of action is explained by a chelating process.

The chelating action of the ethylenediaminotetracetic acid and of its salts with various metal ions is dependent upon temperature, pH, and the specific activity of the chelating agent expressed by the stability constant of the resulting complex.

Its antiviral effect has only been proved in *in vitro* studies on some viral enzymes — the polymerases of viral nucleic acids and the neuraminidase of the A flu virus (Perrin D.D. and Stünzi H., 1981, Pharmac. Ther.22, 255) whose activity was inhibited by the ethylenediaminotetracetic acid thus, viruses multiplication and their penetration into cells was penetrated; however this chelator has not been as yet used in antiviral human therapy.

The prior art does not mention pharmaceuticals or medicinal compositions meant for antiviral therapy with the ethylenediaminotetracetic acid as their active ingredient.

Disclosure of the Invention

The pharmaceutical composition of the present invention consisting essentially of:
a) the calcium disodium salt of ethylenediaminetetracetic acid
b) calcium gluconate and
c) a compound selected from cysteine and reduced glutathione, wherein the weight ratio for the ingredients ranges from 8—12 g to 0.3 to 1 g to 0.05 to 0.2 g respectively per 100 ml of water.

The advantages of the preparation of the invention are the following:

— the drug has a specific curative effect in herpes Zoster, herpes and some other neuroviruses: cranial nerves paralysis, encephalomyelitis, multiple sclerosis, polyradiculoneuritis as well as certain types of dementia, porphyria, Wilson's disease.

— the use of cysteine and the ethylenediaminotetracetic acid in the form of double salt of calcium and sodium as chelating agents provides an anti-inflammatory and trophic and antiviral effect, ensures a rapid diffusion as well as a uniform distribution in the tissues. Since it is not metabolized in the organism, the product is rapidly eliminated (50% within the first hour from i.p. administration and 100% within 24 hours in rats).

— the Na and Ca double salt of the ethylenediaminotetracetic acid and cysteine are not toxic in the dosage prescribed for the above mentioned neurovirotic disorders.

See below 2 concrete examples:

### Example 1

The composition of active ingredients for 100 ml of injectable solution of the invention is the following:

| | |
|---|---|
| Calcium and sodium salt of the ethylenediaminotetracetic acid | 10 g |
| Calcium gluconate | 0.5 g |
| Cysteine hydrochloride | 0.1 g |
| Distilled water | ad 100 ml |

In about 80 ml of fresh distilled water, dissolve, at 80°C, the calcium and sodium salt of the ethylenediaminotetracetic acid and then 0.5 g of calcium gluconate and 0.1 g of cysteine hydrochloride; add distilled water to 100 ml, then filter through a Millipore® filter.

Divide the filtered solution into colourless 10 ml ampoules; sterilize by keeping in an autoclave at 120°C, for 30 minutes.

### Example 2

The composition of active ingredients for 100 ml of injectable solution of the invention is the following:

Calcium and sodium salt of the
ethylenediaminotetracetic acid ............ 10 g
Calcium gluconate ............ 0.5 g
Glutathione (reduced) ............ 0.05 g
Distilled water ............ ad 100 ml

In about 80 ml of fresh distilled water, dissolve at 80°C the calcium and sodium salt of the ethylenediaminotetracetic acid, and then 0.5 g of calcium gluconate, then add the reduced glutathione, previously dissolved in 5 ml of distilled water. Add water to 100 ml and filter through a Millipore® filter.

Divide the filtered solution into colourless 10 ml ampoules; sterilize by keeping in an autoclave at 120°C for 30 minutes.

The injectable solution of the invention should be intramuscularly administered, 10 ml in 24 hours, over a period of 6—7 days.

The hypotheses on the mechanism of action of the pharmaceutical preparation of this invention are based on several recent studies that have proved that the metal ions in the organism are both directly and indirectly involved in the synthesis and metabolism of proteins and viral nucleic acids. The chelation of several such ions by the chelators in this preparation inhibits viruses multiplication through the modification of the metal ions balance.

Besides hindering the process of virus multiplication, the chelators in the claimed pharmaceutical preparation, also act in viral and autoimmune disorders — such as multiple sclerosis whose etiopathology is still unknown; they determine the fluctuation of plasma $Ca^{2+}$, thus restoring certain lesions of the cell membrane and implicity, certain neuronal activities. Consequently, the drug may be also effective in checking such autoaggressive processes.

The association of cysteine with ethylenediaminotetracetic acid has a synergic effect upon the latter's chelating action, since cysteine itself has the ability to fix electrophilic agents (cations) by the thiol group. Moreover, cysteine has the property to form, with some transition metals, hardly soluble complex combinations in which the nitrogen atom is coordinatively linked to the metal.

The initial experiments on the effect of the ethylenediaminotetracetic acid associated with calcium gluconate upon certain neuroviroses have only pointed out a temporary restoring of the nervous influx; the association with cysteine has resulted in a long-lasting remission of paralysis in multiple sclerosis, a strong anti-inflammatory and trophic effect and an intensifying of the antiviral action.

Moreover, cysteine may reduce the mutagenic effect of some chemical compounds (M. Moriya, K. Kato, Y. Shirasu, Mutation Research 1978, 57, 259) and therefore prevents the mutagenic effect of the pharmaceutical preparation.

Reduced glutathione [N-(N-L-γ-glutamyl-L-cysteinyl)glycine] is a natural tripeptide to be found in all animal and vegetable tissues; it is involved in the organism's defence mechanisms and considered to have an anti-mutagenic effect by protecting the nucleophilic groups from informational macromolecules (DNA) against strongly electrophilic agents.

Glutathione capacity to fix through its thiol groups, electrophilic agents (metal cations) may be compared to a chelation mechanism.

Thus, by associating reduced glutathione with the ethylenediaminotetracetic acid, the chelating capacity of the latter is completed and enhanced and the product is provided with an anti-mutagenic action.

In the structure of reduced glutathione, an abnormal peptidic link can be noticed, which is similar to the structure of certain natural very strong antibiotics (produced by bacteria) and which are considered to be endowed with an antiviral property.

Unlike such antibiotics which are highly toxic reduced glutathione, which is a normal multifunctional metabolite of the human organism, is devoid of toxicity.

**Claims**

1. A pharmaceutical composition in the form of an aqueous injectable solution for the treatment of a neuroviroses selected from Herpes Zoster, Herpes Simplex, encephalomyelitis, polyradiculoneuritis, cranial nerve paralysis, or of the autoimmune disease — multiple sclerosis — as well as for the treatment of certain types of dementia, porphyria and Wilson's disease, said composition consisting essentially of:
a) the calcium disodium salt of ethylenediaminetetracetic acid
b) calcium gluconate and
c) a compound selected from cysteine and reduced glutathione, wherein the weight ratio for the ingredients ranges from 8-12 g to 0.3 to 1 g to 0.05 to 0.2 g respectively per 100 ml of water.

2. The use of the composition according to claim 1 for the manufacture of a pharmaceutical composition in the form of an aqueous injectable solution for the treatment of a neuroviroses selected from Herpes Zoster, Herpes Simplex, encephalomyelitis, polyradiculoneuritis, cranial nerve paralysis, or of the autoimmune disease — multiple sclerosis — as well as for the treatment of certain types of dementia, porphyria and Wilson's disease.

3. A pharmaceutical composition in the form of an aqueous injectable solution for the treatment of herpes which consists essentially of
a) the calcium disodium salt of ethylenediaminetetracetic acid
b) calcium gluconate and
c) a compound selected from cysteine and reduced glutathione, wherein the weight ratio for the ingredients ranges from 8-12 g to 0.3 to 1 g to 0.05 to 0.2 g respectively per 100 ml of water.

4. The use of the composition according to one of the claims 1—3 for the manufacture of a pharmaceutical composition in the form of an aqueous injectable solution for the treatment of Herpes.

5. A pharmaceutical composition in the form of an aqueous injectable solution for the treatment of Herpes Zoster which consists essentially of

a) the calcium disodium salt of ethylenediaminetetracetic acid

b) calcium gluconate and

c) a compound selected from cysteine and reduced glutathione, where the weight ratio for the ingredients ranges from 8-12 g to 0.3 to 1 g to 0.05 to 0.2 g respectively per 100 ml of water.

6. The use of the composition according to claim 5 for the manufacture of a pharmaceutical composition in the form of an aqueous injectable solution for the treatment of Herpes Zoster.

**Patentansprüche**

1. Eine pharmazeutische Zusammensetzung in Form einer wässrigen injizierbaren Lösung für die Behandlung einer Neurovirose, ausgewählt aus Herpes Zoster, Herpes Simplex, Encephalomyelitis, Polyradiculoneuritis, cranialer Nerven-Paralyse oder der autoimmunen Krankheit — Multiple Sclerose — als auch für die Behandlung bestimmter Typen von Demenz, Hämotoporphyrie und S. Wilsonscher Krankheit, bestehend im wesentlichen aus:

a) dem Calcium-Dinatrium-Salz der Ethylendiamin-Tretraessigsäure,

b) Calcium-gluconat und

c) einer Verbindung, ausgewählt aus Cystein und reduziertem Glutathion, wobei das Gewichtsverhältnis der Ingredienzien im Bereich von 8 bis 12 g zu 0,3 bis 1 g bzw. zu 0,05 bis 0,2 g pro 100 ml Wasser liegt.

2. Verwendung der Zusammensetzung nach Anspruch 1 zur Herstellung einer pharmazeutischen Zusammensetzung in Form einer wässrigen injizierbaren Lösung für die Behandlung einer Neurovirose, ausgewählt aus Herpes Zoster, Herpes Simplex, Encephalomyelitis, Polyradiculoneuritis, cranialer Nerven-Paralyse oder der autaoimmunen Krankheit — Multiple Sclerose — als auch für die Behandlung bestimmter Typen von Demenz, Hämatoporphyrie und S. Wilsonscher Krankheit.

3. Eine pharmazeutische Zusammensetzung in Form einer wässrigen injizierbaren Lösung für die Behandlung von Herpes, bestehend im wesentlichen aus:

a) dem Calcium-Dinatrium-Salz der Ethylendiamin-Tetraessigsäure,

b) Calcium-gluconat und

c) einer Verbindung, ausgewählt aus Cystein und reduziertem Glutathion, wobei das Gewichtsverhältnis der Ingredienzien im Bereich von 8 bis 12 g zu 0,3 bis 1 g bzw. zu 0,05 bis 0,2 g pro 100 ml Wasser liegt.

4. Verwendung der Zusammensetzung nach einem der Ansprüche 1 bis 3 zur Herstellung einer pharmazeutischen Zusammensetzung in Form einer wässrigen injizierbaren Lösung für die Behandlung von Herpes.

5. Eine pharmazeutische Zusammensetzung in Form einer wärrigen injizierbaren Lösung für die Behandlung von Herpes Zoster, bestehend im wesentlichen aus:

a) dem Calcium-Dinatrium-Salz der Ethylendiamin-Tretraessigsäure,

b) Calcium-gluconat und

c) einer Verbindung, ausgewählt aus Cystein und reduziertem Glutathion, wobei das Gewichtsverhältnis der Ingredenzien im Bereich von 8 bis 12 g zu 0,3 bis 1 g bzw. zu 0,05 bis 0,2 g pro 100 ml Wasser liegt.

6. Verwendung der Zusammensetzung nach Anspruch 5 zur Herstellung einer pharmazeutischen Zusammensetzung in Form einer wässrigen injizierbaren Lösung zur Behandlung von Herpes Zoster.

**Revendications**

1. Composition pharmaceutique sous la forme d'une solution aqueuse injectable pour le traitement d'une neurovirose choisie parmi l'Herpes Zoster, l'Herpès Simplex, l'encéphalomyélite, la polyradiculonèvrite, et la paralysie des nerfs crâniens, ou d'une maladie auto-immune-sclérose multiple- ainsi que pour le traitement de certains types de démence, de porphyrie et de maladie de Wilson, ladite composition comprenant essentiellement:

a) le sel disodique et de calcium de l'acide éthylènediaminetétracétique,

b) du gluconate de calcium et

c) un composé choisie entre la cystéine et le glutathion réduit,

le rapport pondéral de ces ingrédients étant de 8—12 g à 0,3—1 g à 0,05—0,2 g respectivement pour 100 ml d'eau.

2. Utilisation de la composition selon la revendication 1, pour la fabrication d'une composition pharmaceutique sous la forme d'une solution aqueuse injectable pour le traitement d'une neurovirose choisie parmi l'Herpès Zoster, l'Herpès Simplex, l'encéphalomyélite, la polyradiculonévrite, et la paralysie des nerfs crâniens, ou d'une maladie auto-immune-sclérose multiple-ainsi que pour le traitement de certains types de démence, de porphyrie et de maladie de Wilson.

3. Composition pharmaceutique sous la forme d'une solution aqueuse injectable pour le traitement de l'herpès qui comprend essentiellement:

a) le sel disodique et de calcium de l'acide éthylènediaminetétraacétique,

b) du gluconate de calcium et

c) un composé choisi entre la cystéine et le glutathion réduit,

le rapport pondéral de ces ingrédients étant de 8—12 g à 0,3—1 g à 0,05—0,2 g respectivement pour 100 ml d'eau.

4. Utilisation de la composition selon l'une des revendications 1 à 3 pour la fabrication d'une composition pharmaceutique sous la forme d'une solution aqueuse injectable pour le traitement de l'herpès.

5. Composition pharmaceutique sous la forme d'une solution aqueuse injectable pour le traitement de l'Herpès Zoster qui comprend essentiellement:
a) le sel disodique et de calcium de l'acide éthylènediaminetétracétique,
b) du gluconate de calcium et

c) un composé choisi entre la cystéine et le glutathion réduit,
le rapport pondéral de ces ingrédients étant de 8—12 g à 0,3—1 g à 0,05—0,2 g respectivement pour 100 ml d'eau.

6. Utilisation de la composition selon la revendication 5 pour la fabrication d'une composition pharmaceutique sous la forme d'une solution aqueuse injectable pour le traitement de l'Herpès Zoster.